# EUROPEAN PATENT SPECIFICATION

(11) **EP 1 799 562 B1**
(45) Date of publication and mention of the grant of the patent: **20.01.2010**
(21) Application number: 05787956.1
(22) Date of filing: 28.07.2005
(51) Int. Cl.: B65D 5/50, B65D 5/38, A61M 5/00

(54) **Package for holding articles and blank therefor**
Verpackung zum Aufbewahren von Artikeln und Zuschnitt dafür
Emballage pour le stockage d'articles et découpe pour celui-ci

(30) Priority: 28.07.2004 US 591677 P
(43) Date of publication of application: 27.06.2007
(73) Proprietor: MeadWestvaco Corporation, Glen Allen, VA 23060 (US)
(72) Inventor: WESTON, Michael, Chapel Hill, NC 27514 (US); FREEZE, Timothy, Mebane, NC 27302 (US)
(74) Representative: Baldwin, Mark
(86) International application number: PCT/US2005/026779
(87) International publication number: WO 2006/015117

(56) References cited:
- EP-A- 1 002 744
- WO-A-03/101840
- US-A- 2 426 911
- US-A- 5 752 605
- US-A1- 2003 085 262

## Description

### FIELD OF THE INVENTION

This invention relates to packaging, and more specifically to a two-piece apparatus that houses one or more drug delivery devices on an internal slide card within an outer shell, This apparatus may have one or more Internal or external locks that prevent the slide card from being pulled out without triggering a related lock release.

### BACKGROUND OF THE INVENTION

Conventional pharmaceutical packaging has shortcomings with regard to injectables, which create problems for both the manufacturer and end user. For example, there is known to distribute syringes, vials, and parts thereof in packaging that incorporate foam or plastic elements to protect the product. Such conventional packaging normally holds the product in a vertical position. Where conventional packaging holds the product in a horizontal position, the products are typically stacked on top of each other. There is also known to distribute syringes, vials, and part thereof loose - or loose, but individually wrapped - in conventional boxes without any means for holding or protecting the products. US 2003/085262 to Evans discloses a carton assembly having a tray slidable within a carton which carton comprises a body panel and a locking panel spaced apart from the body panel. US 5,752,605 to Cooper discloses a tray and sleeve for protecting packaged articles which tray comprises integral tubular supports having recesses for receiving packaged articles.

The conventional manufacturer that incorporates foam or plastic elements in its packaging to protect the product carries an increased inventory and employs a more complicated manufacturing system to produce its packaging. Further, the conventional manufacturer typically produces one kind of package to be filled by automated means and another kind to be filled by hand, which also increases inventory and the number of product lines.

Conventional manufacturers of injectable holding packaging typically do not provide a child-resistant feature to prevent unauthorized access, or a stopping feature to prevent accidental spillage. Where these features do exist, they exist at the expense of easy access for the end user with limited dexterity. Neither does the known injectable packaging provide ample space to place appropriately sized graphics, such as dose compliance instructions and warnings, for the end user with limited sight.

In addition, conventional manufacturers pack injectables tightly and in the most efficient manner possible -- from the perspective of shipping cost savings -- but, again, at the expense of the end user who has limited physical mobility, such as an end user with arthritis of the fingers. Also conventional manufacturers are known to distribute only wholly-assembled syringes together, parts of syringes together, or vials together, but not whole syringes or parts or vials mixed together. This convention requires the end user to create and maintain an inventory of injectables to fill their individual needs.

End users are familiar with the disposal problems created by the use of injectables. Typically, spent vials, needles, syringes, barrels, and other injectables or parts thereof must be sealed or otherwise protected in order to be disposed of safely. While it is known to dispose of injectables in a separate device, such as a sealable plastic container, there remains a need for an injectable packaging that also serves as a safe means of disposal.

It is apparent from a survey of the pharmaceutical arts that there exists a need for an apparatus that holds and protects all types of drug delivery devices and parts thereof, allows for improved manufacturing processes, includes child-resistant and spill-prevention features, stores a variety of objects in response to the end users' needs, is fitted for easy access by the end user who has limited dexterity, has sufficient area to receive graphics, and provides a means for safe disposal.

### SUMMARY OF THE INVENTION

Generally speaking, the present invention fulfills the needs identified above by providing packaging embodiments comprising an outer sleeve and an inner slide card retained within the outer sleeve and with embodiments that releaseably lock the inner slide card within the outer sleeve. The outer sleeve includes at least one panel with an inner slide card means for locking, an inner slide card means for releasing, and an inner slide card means for stopping. The inner slide card includes a tray and at least one panel configured to cooperatively engage the outer sleeve means for locking, means for releasing, and means for stopping.

In exemplary embodiments the inner slide card means for locking include extension panels or tabs integral to the inner card or, optionally, attachments extending therefrom, configured to releasably engage the outer sleeve. Also the inner card means for releasing includes a catch and a release on an outer sleeve panel, or an attachment extending therefrom, configured to releasably engage said means for locking. The inner slide card means for locking or retaining comprises inner card and outer sleeve extension panels or tabs configured to engage, or attachments or catches associated with the card and sleeve that are configured to engage. Thus the present invention provides a child-resistant feature.

In exemplary embodiments, the inner card means for stopping comprises inner card and outer sleeve extension panels or tabs configured to engage, or attachments or catches associated with the card and sleeve that are configured to engage. Thus the present invention provides a spill-resistant feature to prevent the user from pulling the inner card completely away from the outer sleeve, but which can be opened and closed numerous times to access the drug delivery devices.

Alternative embodiments include an apparatus and method for holding and storing drug delivery devices by providing an inner tray configuration that, by way of example and not limitation, protects a plunger from inadvertent activation; shields a needle from inadvertent exposure; allows easy access to a drug-filled container for removal and replacement; and collects and stores the spent devices. Accordingly, embodiments of the present invention provide an apparatus and system that is able to safely ship drug delivery devices for transepidermal, oral, and hypodermic administration, including pre-filled syringes, needles, vials, ampoules, protective shields, and accessories, safely store the unused devices, and safely store the used devices until all can be safely disposed as a unit.

Alternative embodiments include an apparatus and method for providing compliance directions or information directed to therapy management. In one embodiment, indicia such as, but not limited to, time of day, days of the week, numerical sequence, or dosage amounts are positioned adjacent to the devices. In another embodiment, compliance information or general information related to the medication or therapy is positioned on or with the inner slide card or outer sleeve in a manner easily visible by the user.

Further embodiments include an apparatus for use with a high volume pick-and-pack manufacturing process. The same embodiments provide an apparatus for use with a hand pick-and-pack manufacturing process. Another embodiment includes an apparatus and method for protecting and storing spent drug delivery devices within a secure container until they can be disposed of in a controlled fashion.

Embodiments according to this invention offer at least the following advantages: lightness in weight, resistance to tampering, child-resistance, ease of access, excellent durability, ease of assembly, device protection, ease of storage, ease of disposal, the ability to present devices of different and unusual shapes, and excellent economy.

It is also contemplated that the present invention is not limited to pharmaceutical-related goods, but is applicable to a plethora of delicate, sensitive, or unique portable articles. Small electronic components, jewelry, foods, expensive and precious goods, and any other item which requires a safe, stable, and portable environment in which to be shipped and stored may find an application with the present invention. Other advantages of the present invention will be apparent from the following description, the accompanying drawings, and the appended claims.

### BRIEF DESCRIPTION OF THE DRAWINGS

FIG. 1 is a plan view of an embodiment of a combined slide card and tray blank, according to the present invention.

FIG. 2 is a perspective view of the completely constructed blank of FIG. 1.

FIG. 3 is a perspective view of an alternative embodiment of a slide card and tray, according to the present invention.

FIG. 4 is a plan view of an embodiment of an outer sleeve blank, according to the present invention.

FIG. 5 is a perspective view of the completely constructed blank of FIG. 4.

### DETAILED DESCRIPTION OF THE INVENTION

As required, detailed embodiments of the present invention are disclosed herein. It will be understood that the disclosed embodiments are merely exemplary of the invention that may be embodied in various and alternative forms. The figures are not necessarily to scale, and some features may be exaggerated or minimized to show details of particular components. In other instances, well-known materials or methods have not been described in detail in order to avoid obscuring the present invention. Therefore, specific structural and functional details disclosed herein are not to be interpreted as limiting, but as a basis for the claims and for teaching one skilled in the art to variously employ the present invention.

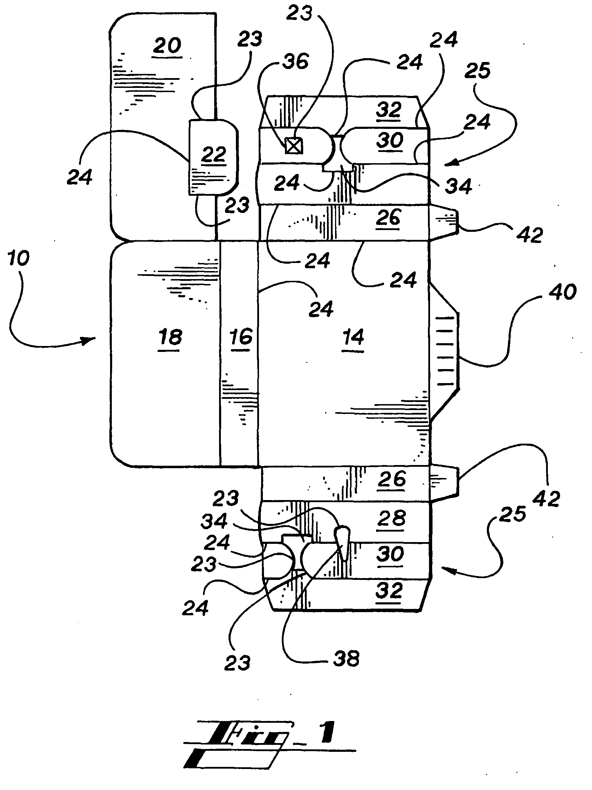

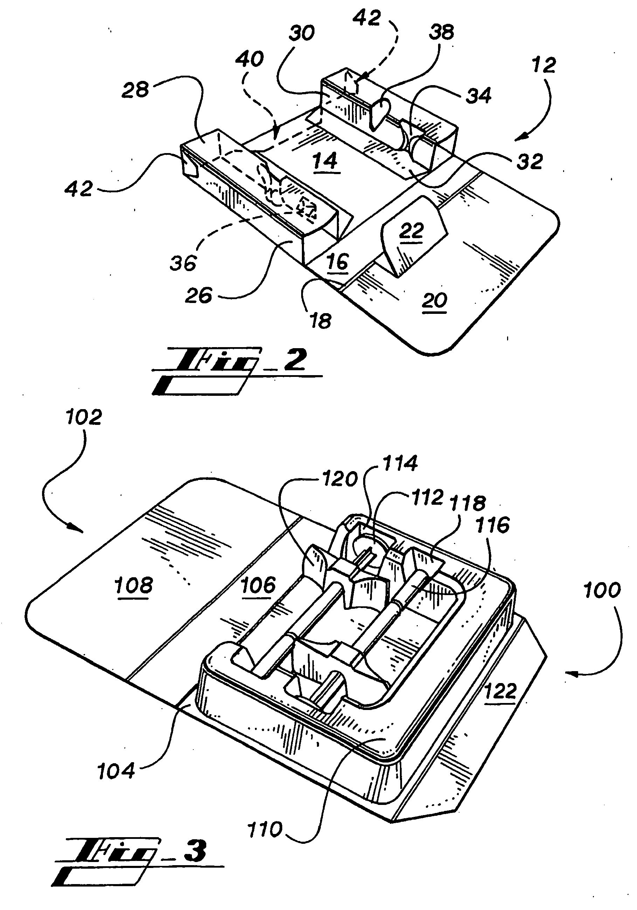

Referring now to the drawings, wherein like numerals represent like features throughout, there are illustrated embodiments of the present invention. Turning first to FIG. 1 and FIG. 2, there is shown an internal slide card blank 10 configured to form an inner tray 12 for holding articles such as drug delivery devices. Herein, the term "drug delivery devices," whether in the singular or plural, is used broadly to refer to all apparatus and parts thereof used in conjunction with transferring solids, fluids, or gases into or out of a body. By way of example and not limitation, a drug delivery device may be in the form of an injectable device comprising a needle, plunger, and cap used by a medical professional to treat a patient with a pharmaceutical drug in liquid form. The same term is applicable herein to refer to all parts of the device as well as the vial(s) used to hold or receive the drug. For purposes of teaching and not limitation, the illustrated embodiments are directed to packaging for articles such as a drug delivery device in the form of an injectable device.

As best shown in FIG. 1, the illustrated blank **10** includes a base panel **14,** spine panel 16, and top panels **18,** 20. The top panel **20** comprises an integral spine support panel **22,** formed by cuts **23** and fold lines **24.** Blank 10 further includes extension panels **25.**

An extension panel **25** comprises an outside sidewall panel **26,** top panel **28,** inside sidewall panel **30,** and securing panel **32.** Further, panel **25** comprises a plunger-receiving slot **34,** formed by cuts **23** and fold lines **24,** and a needle-receiving aperture **36, 38.** Alternative aperture designs are shown to illustrate a means for securing syringe ends with or without a protective cap. As understood by one skilled in the art, in certain embodiments the aperture **36** may be formed by cuts **23** in the form of an "X" while in other embodiments the aperture **38** may be formed by cuts **23** that create a void.

Blank **10** further includes locking tab (first tab) **40** and stopping tabs (second tabs) **42**. As described in detail below, locking tab **40** cooperatively engages with another element to create a child-resistant feature. Also as described below, stopping tabs **42** cooperatively engage with another element to create a pull-out stop that also functions as a spill-resistant feature. With the explanation below, one skilled in the art will understand that the child-resistance feature and stopping feature can both be created, alternatively, with either tabs **40, 42** individually or together. Accordingly, both tabs **40, 42** are a means for locking and a means for stopping.

With respect to assembly, blank **10** is folded and connected, using conventional techniques, to create the combined internal slide card and inner tray **12,** best shown in FIG. 2. One sequence of folding and connecting the blank 10 to form the tray **12,** described merely for the purpose of teaching and not limitation, with reference to the visible side of the illustrated blank **10** as the face and the opposite side as the back, is as follows: The face of top panel **20** is folded and affixed to the face of top panel **18** so that the face of spine support panel **22** overlaps the face of spine panel **16.** The face of each of the outside wall panels **26,** top panel **28,** and inside wall panel 30 are folded toward each other to form an open-end channel. With the faces of panels **26, 28,** and 30 orientated toward each other, the face of securing panel **32** is attached to the face of base panel **14.** In addition, as described below, locking tab **40** will be folded so that the back of locking tab **40** is orientated toward the back of base panel **14.** Similarly, stopping tabs **42** will be folded so that the backs of stopping tabs **42** are orientated toward the backs of the respectively adjacent outside sidewall panels **26.**

After assembly, the inner tray **12** is configured to receive and store an injectable device (not shown). In the illustrated embodiment of FIG. 2, a plunger handle may be received and secured by the plunger-receiving slot **34** while a needle may be received and secured by the needle-receiving apertures **36, 38.** By way of illustration and not limitation, the deceiving slot **34** and cutouts **36, 38** include features for securing the injectables. Here, the receiving slot **34** is shaped as an hourglass because this shape holds a plunger handle in a particular position while allowing easy access. Those skilled in the art will understand that receiving slot **34,** as a means for securing an injectable, may be configured in various shapes, depending on the injectable and ease or complexity of access desired. For example, a receiving slot **34** in the shape of a "J," "L," "G," and "H" all provide varying levels of security and access for the injectable. In addition, the means for securing may comprise inserts of different materials, such as plastic or rubber yokes, to further secure or removably lock the injectables in position.

Similarly, here apertures **36, 38** are shaped as an "X" and as an oval because these shapes can hold a bare needle, capped needle, or the neck of a vial in a particular position while allowing easy access. Those skilled in the art will understand that apertures **36, 38,** as a means for securing an injectable, may be configured in various shapes depending on the injectable and ease or complexity of access desired. For example, an injectable comprising a plunger handle, barrel, and needle may be stored by placing the plunger handle in receiving slot **34** and the needle in the aperture **36, 38** located in the opposite inside sidewall panel **30,** with the barrel spanning the space in between. In this manner the plunger of a pre-filled injectable is protected from inadvertent pressure, and the user is protected from inadvertent contact with the needle.

FIG. 3 shows an alternative embodiment of an inner card **100** for receiving and holding injectables, according to the present invention. The illustrated inner card **100** includes an internal slide **102** that comprises a base panel **104,** spine panel **106,** and top panel **108.** Here, rather than using paperboard to monolithically form the inner tray and slide card as described above, the inner tray **110** is thermoformed separately and then affixed to the slide card **102.** The inner tray **110** comprises a means for securing and holding injectables, such as the plunger **112** in the plunger-receiving recess **114** and the barrel **116** in the barrel-receiving recess **118.** The recesses **114, 118** may be configured to lock in or otherwise secure the injectable by including a means for resisting removal of the injectable, such as fold-over locking flaps, indentions, or inserts. Accordingly, a means for holding and storing a drug delivery device includes a tray constructed in a variety of ways.

Here the inner tray **110** is also configured to allow for easy access to the injectables. By way of illustration and not limitation the injectables are arranged alternately so that the end user, who may have limited physical mobility such as that resulting from arthritis, can retrieve one injectable without affecting another. As illustrated, purposefully orientating the widest portion of the injectable, in this example the finger guard **120,** to take the most space to provide the greatest accessibility is a desirable feature of this embodiment. Such horizontal orientating also provides easy viewing of the products so the user may easily distinguish between them. Further, such orientating provides ample area to receive graphics. Patient and healthcare provider information, such as dose compliance, can be made easily visible to the user.

The embodiment of FIG. 3 further includes tab **122** which may function as a means for locking and/or as a means for stopping, like the locking tab **40** and stopping tab **42** described herein.

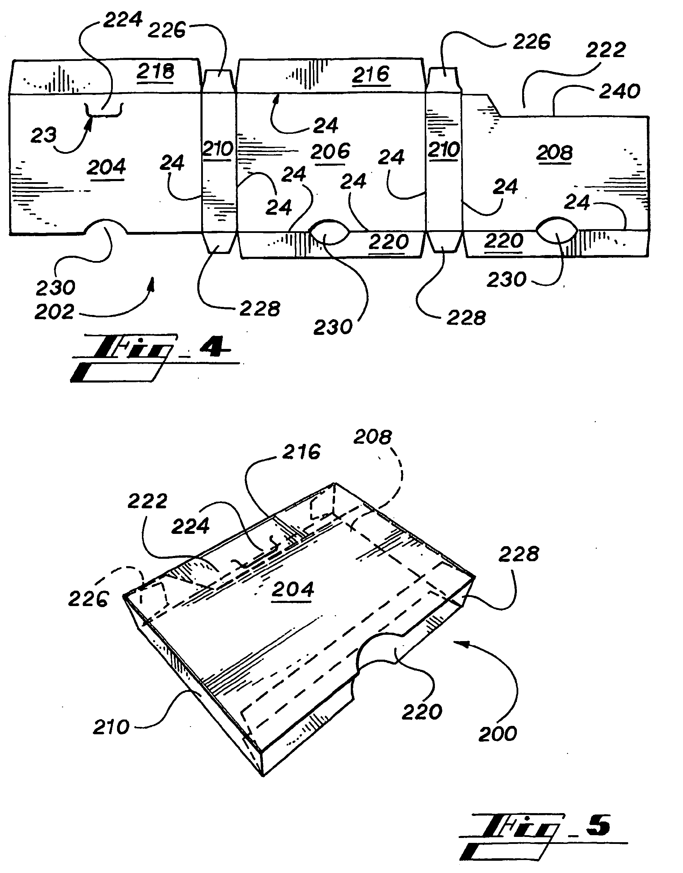

Turning now to FIGS. 4 and 5, there is shown an outer sleeve 200, for receiving an inner card **12, 100** and the related outer sleeve blank **202.** As best shown in FIG. 4, the illustrated blank **202** includes side panels **204, 206, 208,** spine panels (sidewall panels) **210,** end panels **216, 218,** and extension panels **220.**

With regard to assembly, the blank **202** is folded and connected, using conventional techniques, to create the outer sleeve **200,** best shown in FIG. 5 as a slip case. One sequence of folding and connecting the blank **202** is as follows, with reference to the visible side of the illustrated blank **202** as the face and the opposite side as the back: Side panel **204** is folded along fold lines **24** under the side panels **206, 208** and positioned over panel **208** so that the back of panel **204** may be affixed to the face of panel **208.** In this embodiment, panel **204** is overlayed and affixed to panel **208** so that the cutout **222** of panel **208** surrounds the release button **224.** In other words, the release button **224** is unobstructed by panel **208.**

Tabs **226** are folded inwardly to create a closed endwall, such that the backs of tabs **226** are orientated toward the interior case created by the side panels **204, 206** and spine panels **210.** End panels **216, 218** are then folded inwardly so that the face of tabs **226** may be affixed to the back of panel **216** and the face of panel **216** may be affixed to the back of panel **218,** to complete the closed endwall.

In addition, extension panels **220** are folded inwardly so that the back of panels **220** may be affixed to the backs of respectively adjacent side panels **206, 208.** Similarly, extension tabs (third tabs) **228** are folded inwardly so that the backs of tab **228** may be affixed to the respectively adjacent spine panels **210.** The folding of panels **220** form finger-access areas at the cutouts **230.**

Generally speaking, injectables are placed within inner tray **12, 100** and then the inner tray **12, 100** is inserted into outer sleeve **200.** The apparatus holds and protects the injectables until they are retrieved for use. In practice, and with reference to FIGS. 1 and 2, injectables are placed within the inner tray **12** and then several panels or tabs are folded before the internal card and inner tray **12** is inserted into the outer sleeve **200.** For purposes of teaching and not limitation, the following folding sequence is described. Top panel **20** is folded so as to cover the injectables and orientate the spine support panel **22** so as to provide support for the spine **16.** In the illustrated embodiment, the back of top panel **20** is now adjacent to the injectables and panel **22** is now substantially parallel to panel **16.** Further, locking tab **40** is folded outwardly, so that the back of tab **40** is close to or touching the back of base panel **14** and stopping tabs **42** are folded outwardly so that the backs of tabs **42** are close to or touching the back of the respectively adjacent sidewall panel **26.**

With the internal card and inner tray **12** loaded with injectables and folded as described immediately above, the tray **12** is inserted, starting with the edge comprising the tabs **40, 42** and with tab **40** receivingly aligned with the catch formed by cutout **222** and release button **224,** into the void of outer sleeve **200.** The internal card and inner tray **12** is fully inserted into the outer sleeve **200,** to a fully closed position. As understood by those skilled in the art, the spring tension created by the outwardly folded tabs **40, 42** causes the leading edge of the tabs **40, 42** to press against the interior side of the panels **204, 208, 210.** The position of the tab **40** provides a locking feature and the tab(s) **42** provide a stopping feature. For purposes of teaching and not limitation, the locking feature is described with regard to tab **40,** and the stopping feature is described with regard to tab **42.** It will be understood that either tabs **40** or **42** could interchangeably perform either the locking or stopping features.

In this illustration the locking feature includes the catch formed by the cutout **222,** the release button **224** and cooperatively interlocking tab **40.** The spring tension created by the compressed tab **40** causes the leading edge of tab **40** to engage the internal edge **240** of the panel **208.** With the tab **40** and leading edge **240** engaged, the inner tray **12** is locked and cannot be opened. This means for locking creates a child-resistant feature. To unlock the child-resistant feature of this embodiment and thereby open the tray **12,** the user depresses the release button **224,** created by the cut **23,** which in turn depresses the tab **40** to disengage the leading edge of the tab **40** from the internal edge **240.** It will be understood that another means for locking may be created by placing one or more release buttons in panel(s) **210** to releaseably engage tab(s) **42.**

After releasing the optional locking feature, the inner card **12** may be pulled out, until the stopping tabs **42** engage the extension tabs **228,** to a fully open position. As will be understood by those skilled in the art, the spring tension created by the compressed tabs **42** causes the leading edge of the tabs **42** to engage the tabs **228.** Once engaged, the tray **12** cannot be further removed from the outer sleeve **200** but may be reinserted to a fully closed position if desired. In this manner, tabs **42, 228** act as a stopping device to prevent inner card **12** from being pulled completely out of outer sleeve **100.** It will be understood that another means for stopping is created by allowing tab 40 to engage extension tabs **220.**

The user may open and close the apparatus by withdrawing and replacing the inner tray **12** within the outer sleeve **200** as often as desired. In the fully open position, the user may fold back the top panel **20** to access an injectable. After accessing the desired injectable, the user replaces the top panel **20** and reinserts the inner tray **12** within the outer sleeve **200** for future use.

Alternatively, an embodiment designed to be disposed of, together with used injectables, may be placed within a red plastic bag (not shown but provided with the embodiment) thereby giving notice of the contents. By way of illustration and not limitation, additional means for protecting and sealing an embodiment to be disposed of, together with used injectables, include sealable bags, a self-sealing outer sleeve, a sealable outer sleeve large enough to receive the inner tray **12** and outer sleeve **200.** Similarly, taping the inner tray **12** within the outer sleeve **200** with red tape giving notice of the contents is a means for protecting and sealing.

With regard to the materials of construction, the illustrated embodiments comprise paperboard as a substrate for blanks **10, 202,** which is typically constructed from a sheet of bleached sulphate, solid unbleached sulphate, or clay-coated newsback. Compositionally the paperboard coating is a fluidized blend of materials, such as coating clay, calcium carbonate, and/or titanium dioxide, with starch or adhesive that is smoothly applied to the traveling surface. Successive densification and polishing finish the mineral-coated surface to a superior, graphic-print surface. Other embodiments may comprise vacuum-formed plastic or paper, press-formed paperboard, cardboard, or combinations thereof.

The above-described embodiments are merely exemplary illustrations of implementations set forth for a clear understanding of the principles of the invention. Variations, modifications, and combinations may be made to the above-described embodiments without departing from the scope of the claims. All such variations, modifications, and combinations are included herein by the scope of this disclosure and the following claims.

## Claims

1. A package for holding articles, comprising a slide card (12, 100) and outer sleeve (200), said slide card comprising:
a base panel (14) configured to mount an article, a first tab (40) hinged to the base panel for engaging with a catch (222) associated with the outer sleeve and a first extension panel (25) hinged to the base panel along a first side, wherein said first extension panel comprises an outside sidewall panel (26) and a second tab (42) hinged to the outside sidewall panel,
wherein said outer sleeve (200) comprises:
an open end,
a first panel (204), a second panel (206) opposite the first panel, opposing sidewall panels (210) connected to the first and second panels, an end wall (216, 218) connected to each of the first and second panels or the sidewalls, an extension panel (220) hinged to one of said first or second panel (204, 206) and a third tab (228) hinged to one of the opposing sidewalls panels (210),
wherein the first and second panels and side and end walls define a void that includes said catch (222) configured to receive and releasably engage said first tab (40) of the slide card (12, 100) for locking the slide card within the outer sleeve (200) and wherein said first tab (40) engages with said extension panel (220) to form a first stopping mechanism and wherein the second and third tabs (42, 228) cooperate to form a second stopping mechanism for preventing removal of the slide card from the open-end of the outer sleeve.

2. The package of claim 1, wherein the outer sleeve (200) further includes a release (224) configured to disengage the catch (222).

3. The package of claim 1, wherein the slide card (12, 100) further comprises at least one article securing element (34, 38).

4. The package of claim 1, further comprising a spine panel (16) hingedly connected to the base panel (14).

5. The package of claim 4, further comprising a top cover (18) hingedly connected to the spine panel (16) and configured to cover at least a portion of the slide card.

6. A blank (10) for forming an integrated slide card and tray (12) comprising:
a base panel (14);
a first extension panel (25) hingedly connected to the base panel along a first side;
a second extension panel (25) hingedly connected to the base panel along a second side; and
a first tab (40) hingedly connected to the base panel along a third side;
wherein the extension panels (25) are configured to be folded and connected to form the sidewalls of a slidable tray, said slidable tray being configureable to insert within an outer sleeve (200)
having an open end, the first tab (40) being configured to engage a catch (222) associated with said outer sleeve, thereby forming a locking mechanism, and to engage with an extension panel (220) associated with said outer sleeve for forming a first stopping mechanism,
wherein each extension panel (25) further comprises:
an outside sidewall panel (26);
a top panel (28);
an inside sidewall panel (30); and
a securing panel (32);
wherein the top and sidewall panels are configured to be folded and the securing panel is configured to be secured to the base panel to form an open-channel sidewall in a setup condition;
wherein the open channel sidewall further comprises a second tab (42) configureable to engage a third tab (228) associated with the outer sleeve (200) for forming a second stopping mechanism.

7. The blank of claim 6, further comprising a spine panel (16) hingedly connected to the base panel (14) hand to a first cover panel (18).

8. The blank of claim 7, wherein the cover panel (18) is hingedly connected to a second cover panel (20) that comprises a spine support panel (22).

9. The blank of any of claims 6 to 8, wherein the open-channel sidewall further includes at least one article receiving element (36, 38).

## Patentansprüche

1. Verpackung zum Halten von Gegenständen, umfassend eine Einschubkarte (12, 100) und eine Außenhülse (200), wobei die Einschubkarte umfasst:
eine Bodenwandfläche (14), die ausgestaltet ist, einen Gegenstand aufzunehmen, eine erste Lasche (40), die an die Bodenwandfläche angelenkt ist, um eine Sperreinrichtung (222) in Eingriff zu nehmen, die mit der Außenhülse im Zusammenhang steht, sowie eine erste Verlängerungswandfläche (25), die entlang einer ersten Seite an die Bodenwandfläche angelenkt ist,
wobei die erste Verlängerungswandfläche eine Außenseitenwandfläche (26) und eine zweite Lasche (42) umfasst, die an die Außenseitenwandfläche angelenkt ist,
wobei die Außenhülse (200) umfasst:
ein offenes Ende,
eine erste Wandfläche (204), eine zweite Wandfläche (206) gegenüber der ersten Wandfläche, gegenüberliegende Seitenwandflächen (210), die mit der ersten und der zweiten Wandfläche verbunden sind, eine Endwand (216, 218), die mit jeder der ersten und zweiten Wandflächen oder den Seitenwänden verbunden ist, eine Verlängerungswandfläche (220), die an die erste oder die zweite Wandfläche (204, 206) angelenkt ist, sowie eine dritte Lasche (228), die an eine der gegenüberliegenden Seitenwandflächen (210) angelenkt ist,
wobei die erste und die zweite Wandfläche und die Seiten- und Endwände einen Hohlraum definieren, der die Sperreinrichtung (222) einschließt, die dazu ausgestaltet ist, die erste Lasche (40) der Einschubkarte (12, 100) aufzunehmen und freigebbar in Eingriff zu nehmen, um die Einschubkarte innerhalb der Außenhülse (200) zu verriegeln, und wobei die erste Lasche (40) die Verlängerungswandfläche (220) in Eingriff nimmt, um einen ersten Stoppmechanismus auszubilden, und wobei die zweite und die dritte Lasche (42, 228) zusammenwirken, um einen zweiten Stoppmechanismus auszubilden, um das Entfernen der Einschubkarte aus dem offenen Ende der Außenhülse zu verhindern.

2. Verpackung nach Anspruch 1, wobei die Außenhülse (200) ferner eine Freigabeeinrichtung (224) umfasst, die dazu ausgestaltet ist, die Sperreinrichtung (222) zu entsperren.

3. Verpackung nach Anspruch 1, wobei die Einschubkarte (12, 100) ferner wenigstens ein Gegenstandsbefestigungselement (34, 38) umfasst.

4. Verpackung nach Anspruch 1, wobei die Verpackung ferner eine Buchrückenwandfläche (16) umfasst, die gelenkig mit der Bodenwandfläche (14) verbunden ist.

5. Verpackung nach Anspruch 4, wobei die Verpackung ferner eine obere Abdeckung (18) umfasst, die gelenkig mit der Buchrückenwandfläche (16) verbunden ist und dazu ausgestaltet ist, wenigstens einen Abschnitt der Einschubkarte abzudecken.

6. Zuschnitt (10) zum Ausbilden einer integrierten Kombination aus Einschubkarte und Schale (12), umfassend:
eine Bodenwandfläche (14);
eine erste Verlängerungswandfläche (25), die entlang einer ersten Seite gelenkig mit der Bodenwandfläche verbunden ist;
eine zweite Verlängerungswandfläche (25), die entlang einer zweiten Seite gelenkig mit der Bodenwandfläche verbunden ist; und eine erste Lasche (40), die entlang einer dritten Seite gelenkig mit der Bodenwandfläche verbunden ist;
wobei die Verlängerungswandflächen (25) dazu ausgestaltet sind, gefaltet und verbunden zu werden, um die Seitenwände einer schiebbaren Schale auszubilden, wobei die schiebbare Schale ausgestaltet werden kann, in eine Außenhülle (200) eingebracht zu werden, die ein offenes Ende aufweist, wobei die erste Lasche (40) dazu ausgestaltet ist, eine Sperreinrichtung (222) in Eingriff zu nehmen, die mit der Außenhülse im Zusammenhang steht, um somit einen Verriegelungsmechanismus auszubilden, sowie um eine Verlängerungswandfläche (220) in Eingriff zu nehmen, die mit der Außenhülse im Zusammenhang steht, um einen ersten Stoppmechanismus auszubilden,
wobei jede Verlängerungswandfläche (25) ferner umfasst:
eine Außenseitenwandfläche (26);
eine Deckenfläche (28);
eine Innenseitenwandfläche (30); und
eine Befestigungswandfläche (32);
wobei die Deckenwandfläche und die Seitenwandflächen dazu ausgestaltet sind, gefaltet zu werden, und die Befestigungswandfläche dazu ausgestaltet ist, an die Bodenwandfläche befestigt zu werden, um eine Offenkanal-Seitenwand in einem aufgerichteten Zustand auszubilden;
wobei die Offenkanal-Seitenwand ferner eine zweite Lasche (42) umfasst, die ausgestaltet werden kann, eine dritte Lasche (228) in Eingriff zu nehmen, die mit der Außenhülse (200) im Zusammenhang steht, um einen zweiten Stoppmechanismus auszubilden.

7. Zuschnitt nach Anspruch 6, wobei der Zuschnitt ferner eine Bruchrückenwandfläche (16) umfasst, die gelenkig mit der Bodenwandfläche (14) und mit einer ersten Abdeckungswandfläche (18) verbunden ist.

8. Zuschnitt nach Anspruch 7, wobei die Abdeckungswandfläche (18) gelenkig mit einer zweiten Abdeckungswandfläche (20) verbunden ist, die eine Buchrückenunterstützungswandfläche (22) umfasst.

9. Zuschnitt nach einem der Ansprüche 6 bis 8, wobei die Offenkanal-Seitenwand ferner wenigstens ein Gegenstandsaufnahmeelement (36, 38) umfasst.

## Revendications

1. Emballage destiné à retenir des articles, comprenant une carte coulissante (12, 100) et un manchon externe (200), ladite carte coulissante comprend:
un panneau de base (14) configuré pour fixer un article, une première languette (40) articulée sur le panneau de base pour pouvoir venir en contact avec un élément de fermeture (222) associé au manchon externe et un premier panneau d'extension (25) articulé sur le panneau de base le long d'un premier côté, dans lequel ledit premier panneau d'extension (25) comprend un panneau de paroi latérale extérieur (26) et une deuxième languette (42) articulée sur le panneau de paroi latérale extérieur,
dans lequel ledit manchon externe (200) comprend:
une extrémité ouverte,
un premier panneau (204), un second panneau (206) placé en regard du premier panneau, des panneaux de paroi latérale opposés (210) connectés aux premier et second panneaux, une paroi d'extrémité (216, 218) connectée à chacun des premier et second panneaux ou des parois latérales, un panneau d'extension (220) articulé sur un desdits premier et second panneaux (204, 206) et une troisième languette (228) articulée sur un des panneaux de paroi latérale opposés (210),
dans lequel les premier et second panneaux et les parois latérales et d'extrémité délimitent une cavité qui comporte ledit élément de fermeture (222) configuré pour recevoir et venir en prise de manière détachable avec ladite première languette (40) de la carte coulissante (12, 100) afin de pouvoir verrouiller la carte coulissante dans le manchon externe (200) et dans lequel ladite première languette (40) vient en prise avec ledit panneau d'extension (220) pour former un premier mécanisme de blocage et dans lequel les deuxième et troisième languettes (42, 228) coopèrent de manière à former un second mécanisme de blocage, destiné à empêcher le retrait de la carte coulissante de l'extrémité ouverte du manchon externe.

2. Emballage selon la revendication 1, dans lequel le manchon externe (200) comporte en outre un élément de déblocage (224) configuré de manière à pouvoir se désaccoupler de l'élément de fermeture (222).

3. Emballage selon la revendication 1, dans lequel la carte coulissante (12, 100) comprend en outre au moins un élément de fixation d'articles (34, 38).

4. Emballage selon la revendication 1, comprenant en outre un panneau de dos (16) relié de manière articulée au panneau de base (14).

5. Emballage selon la revendication 4, comprenant en outre une couverture supérieure (18) reliée de manière articulée au panneau de dos (16) et configuré de manière à couvrir au moins une partie de la carte coulissante.

6. Découpe (10) destinée à former une carte coulissante intégrée et un plateau (12) comprenant:
un panneau de base (14)
un premier panneau d'extension (25) connecté de manière articulée au panneau de base le long d'un premier côté;
un second panneau d'extension (25) connecté de manière articulée au panneau de base le long d'un deuxième côté; et
une première languette (40) connectée de manière articulée au panneau de base le long d'un troisième côté;
dans laquelle les panneaux d'extension (25) sont configurés de manière à pouvoir être pliés et connectés pour former les parois latérales d'un plateau coulissant, ledit plateau coulissant étant configurable pour être inséré dans le manchon externe (200) présentant une extrémité ouverte, la première languette (40) étant configurée pour pouvoir venir en prise avec un élément de fermeture (222), associé au dit manchon externe, et de cette manière formant un mécanisme de verrouillage, ainsi que pour pouvoir venir en prise avec un panneau d'extension (220) associé au dit manchon externe pour former un premier mécanisme de blocage,
dans laquelle chaque panneau d'extension (25) comprend en outre:
un panneau de paroi latérale extérieur (26);
un panneau supérieur (28);
un panneau de paroi latérale interne (30); et
un panneau de fixation (32);
dans laquelle les panneaux supérieur et de paroi latérale sont configurés de manière à pouvoir être pliés et, une fois assemblé, le panneau de fixation est configuré de manière à pouvoir être fixé au panneau de base afin de pouvoir former une paroi de canal ouvert ;
dans laquelle la paroi latérale de canal ouvert comprend en outre une deuxième languette (42) configurable de manière à pouvoir venir en prise avec une troisième languette (228) associée à un manchon externe (200) pour former un second mécanisme de blocage.

7. Découpe selon la revendication 6, comprenant en outre un panneau de dos (16) relié de manière articulée au panneau de base (14) et au premier panneau de couverture (18).

8. Découpe selon la revendication 7, comprenant en outre un panneau de couverture (18) relié de manière articulée au second panneau de couverture (20), lequel comprend un panneau de support de dos (22).

9. Découpe selon l'une quelconque des revendications 6 à 8, dans laquelle la paroi latérale de canal ouvert comporte en outre au moins un élément de réception d'articles (36, 38).
